# EUROPEAN PATENT APPLICATION

(11) **EP 2 335 488 A1**
(43) Date of publication of application: **22.06.2011**
(21) Application number: 10183941.3
(22) Date of filing: 12.07.2002
(51) Int. Cl.: A23C 9/00, G01N 33/06

(54) **Populations of dairy cows producing milk with desirable characteristics and methods of making and using same**

(30) Priority: 16.07.2001 NZ 51300401
(62) Divisional of application: 02763130.8
(71) Applicant: Therapeutic Foods Limited, Cambridge 3494 (NZ)
(72) Inventor: Cooper, Garth, Ponsonby (NZ)
(74) Representative: Hayes, Adrian Chetwynd

(57) **Abstract**

A method of producing MFAC milk from bovine cows capable of producing MFAC milk when fed a conventional diet, the method including the steps of:
(i) identifying bovine cows capable of producing MFAC milk when fed a conventional diet;
(ii) selecting cows capable of producing MFAC milk when fed a conventional diet; and
(iii) milking the selected cows to obtain the MFAC milk;

where the MFAC milk comprises:
(A) less than about 60% total saturated fat, at least about 30% monounsaturated fatty acids and at least about 9% total polyunsaturated fatty acids; or
(B) less than about 60% total saturated fat, at least about 30% monounsaturated fatty acids, at least about 6% total polyunsaturated fatty acids and less than about 20% palmitic (16:0) acid; or
(C) less than about 60% total saturated fat, at least about 30% monounsaturated fatty acids, at least about 6% total polyunsaturated fatty acids and at least about 5% linoleic (18:2) acid.

## Description

### BACKGROUND OF THE INVENTION

High fat diets, particularly those high in saturated fats, have long been shown to have adverse effects on cardiovascular disease (CVD) risk factors such as serum total- and LDL-cholesterol (Grundy & Vega, 1988, Am. J. Clin. Nutr. 47:822). For many years the recommendation to replace dietary saturated fats with carbohydrates has been an important public health message both for weight loss and improvements in cardiovascular health *per se* (National Institutes of Health, "Clinical guidelines-- the evidence report" (1998)). However this has been questioned and considerable controversy has arisen (Katan et al, 1997, Am. J. Clin. Nutr. 61 (6 Suppl.) 136S). Whilst rigorously controlled, residential trials of well-motivated compliant participants have clearly shown that a low-fat high-CHO diet can result in weight loss (Prewitt et al., 1991, Am. J. Clin. Nutr. 54:304; Stubbs et al., 1995, Am. J. Clin. Nutr. 62:316; Poppitt et al., 1998, Am. J. Clin. Nutr. 68:1012), in larger, longer-term community trials the results have been predominantly (Sheppard et al.,1991, Am. J. Clin. Nutr. 54:821; Jeffrey et al.,1995, Int. J. Obesity 19:132; Willett, 1998, Am. J. Clin. Nutr. 67 (Suppl.):556S) although not entirely (Saris et al., 2000, Int. J. Obesity 24:1310; Poppitt et al., 2001, Am. J. Clin. Nutr, in press) disappointing. Of equal concern are the purported adverse effects on circulating lipids. Whilst the replacement of saturated fat by CHO is well established in reducing circulating LDL-cholesterol, it may be accompanied by a concomitant reduction in HDL-cholesterol and/or increase in serum triacylglycerol (TG), both adverse factors for cardiovascular disease risk (Katan et al., 1997, New Engl. J. Med. 337:562; Katan ,1998, Am. J. Clin. Nutr. 67 (Suppl.)573S).

An alternate approach to improving cardiovascular risk is to make alterations in the quality of the fat consumed. Many trials have shown that replacement of dietary saturated fatty acids with predominantly mono- (MUFA) and/or polyunsaturated (PUFA) fatty acids can improve lipid profile considerably (Grundy & Vega, *supra ,* Berry et al., 1991, Am. J. Clin. Nutr. 53:899; Hu et al., 1997, New Engl J. Med. 337:1491), possibly by increasing the activity of LDL receptors in the liver. Most studies have investigated extreme manipulations of diet. Strategies in which saturated fatty acids are be replaced by MUFAs or PUFAs within a normal diet would be of considerable importance to public health policy if it could be shown that significant reductions in risk could be achieved through simple physiological changes in commonly eaten foods. One of the most important food groups known to be naturally high in saturates, particularly myristic and palmitic acids, are the dairy fats. Dairy products comprise a considerable proportion of the diet in countries such as the United States, Europe and New Zealand and thus make an excellent tool through which reductions in adverse lipid and lipoprotein profiles may possibly be achieved.

### SUMMARY OF THE INVENTION

The present inventors have discovered that certain individual cows produce milk with relatively low levels of saturated fats and relatively high levels of monounsaturated and polyunsaturated fatty acids.

In one aspect, the invention provides a population of cows wherein substantially all of the milk-producing cows in the population produce milk comprising less than about 60% total saturated fat, at least about 30% mono-unsaturated fatty acids (hereinafter, "MUFA"), and at least about 9% total poly-unsaturated fatty acids (hereinafter, "PUFA"). In a related embodiment, the milk comprises less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2. The cows may be fed a conventional diet. In an embodiment, the cow population comprises at least 10 milk-producing cows. In still other embodiments, at least one of the cows is a Friesian, Guernsey, Holstein, Ayreshire, Jersey, Brown Swiss, or Milking Shorthorn.

In another aspect, the invention provides a method of generating a population of cows wherein substantially all of the milk-producing cows in the population produce MFAC milk, said method comprising: (a) obtaining a milk sample produced by an individual cow; (b) determining whether the fat composition of the milk sample is characteristic of a MFAC milk; (c) identifying an individual cow that produced a milk sample with a fat composition characteristic of a MFAC milk as a milk-producing cow that produces MFAC milk; (d) repeating steps (a) to (c) with additional individual cows until a plurality of cows are identified as milk-producing cows that produce MFAC milk; and, (e) physically or informationally segregating the plurality of cows, thereby generating a cow population wherein substantially all of the milk-producing cows in the population produce MFAC milk. In a related embodiment, the fat composition characteristic of a MFAC milk is less than about 60% total saturated fat, at least about 30% mono-unsaturated fatty acids, and at least about 9% total poly-unsaturated fatty acids. In another embodiment, the fat composition characteristic of a MFAC milk is less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2. In an embodiment, the plurality of cows is at least 10 cows.

In another aspect, the invention provides a population of cows generated by the method of generating a population of cows wherein substantially all of the milk-producing cows in the population produce MFAC milk, as described above. In another aspect, the invention provides progeny of a cow in the population.

In another aspect, the invention provides a method for breeding cattle to generate progeny cows that produce MFAC milk, said method comprising: (a) identifying at least one cow that, when fed a conventional diet, produces milk with a fat composition characteristic of a MFAC milk; (b) breeding the cow to produce progeny; and, (c) selecting progeny that produce milk with a milk fat composition characteristic of a MFAC milk. In a related embodiment, the fat composition characteristic of a MFAC milk is less than about 60% total saturated fat, at least about 30% mono-unsaturated fatty acids, and at least about 9% total poly-unsaturated fatty acids.

In another aspect, the invention provides a population of cows produced according to the method for breeding cattle to generate progeny cows that produce MFAC milk, described above, wherein substantially all of the milk-producing cows in the population produce milk comprising less than about 60% total saturated fat, at least about 30% mono-unsaturated fatty acids (MUFA), and at least about 9% total poly-unsaturated fatty acids (PUFA) when fed a conventional diet. In another aspect, the invention provides progeny of a cow in the population.

In another aspect, the invention provides a pooled milk composition comprising milk from a plurality of individual cows capable of producing MFAC milk when fed a conventional diet. In a related embodiment, the plurality comprises at least 10 cows. In another related embodiment, the composition does not contain milk from cows that do not produce MFAC milk. In another aspect, the invention provides a milk-based product made using the pooled milk compositions. In various embodiments, the milk-based product is powdered milk, condensed milk, skim milk, cream, butter, cheese, chocolate, ice cream, yogurt or infant-formula.

In another aspect, the invention provides a pooled milk fat composition comprising milk from a plurality of individual cows fed conventional diets, wherein the pooled milk composition possesses a fat composition characteristic of the fat composition of a MFAC milk. In another aspect the invention provides a milk-based product made using the pooled milk composition. In various embodiments, the milk-based product is powdered milk, condensed milk, skim milk, cream, butter, cheese, chocolate, ice cream, yogurt or infant-formula.

In another aspect, the invention provides a method of identifying an individual milk-producing cow that produces MFAC milk comprising: (a) obtaining a milk sample produced by an individual cow that has been fed a conventional diet for at least about three days prior to the time the sample is obtained; (b) determining whether the fat composition of the milk sample is characteristic of a MFAC milk; and, (c) identifying an individual cow that produced a milk sample with a fat composition characteristic of a MFAC milk as a milk-producing cow that produces MFAC milk. In another embodiment, the method further comprises repeating steps (a) to (c) with additional individual cows until a plurality of cows are identified as milk-producing cows that produce MFAC milk. In another embodiment, the method further comprises physically or informationally segregating the plurality of cows, thereby generating a cow population wherein substantially all of the milk-producing cows in the population produce MFAC milk. In a related embodiment, the plurality of cows comprises at least 5 cows. In related embodiments, the fat composition characteristic of a MFAC milk is less than about 60% total saturated fat, at least about 30% mono-unsaturated fatty acids, and at least about 9% total poly-unsaturated fatty acids. In another aspect, the fat composition characteristic of a MFAC milk is less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2.

In another aspect, the invention provides a method of identifying an individual cow capable of producing MFAC milk, said method comprising: (a) identifying a genetic marker in bovines associated with the phenotype in milk-producing cows of producing MFAC milk; (b) obtaining a nucleic acid sample from an individual cow; and, (c) detecting the presence of the genetic marker in the nucleic acid, thereby identifying the identifying the cow as an a cow capable of producing MFAC milk.

### BRIEF DESCRIPTION OF THE FIGURES

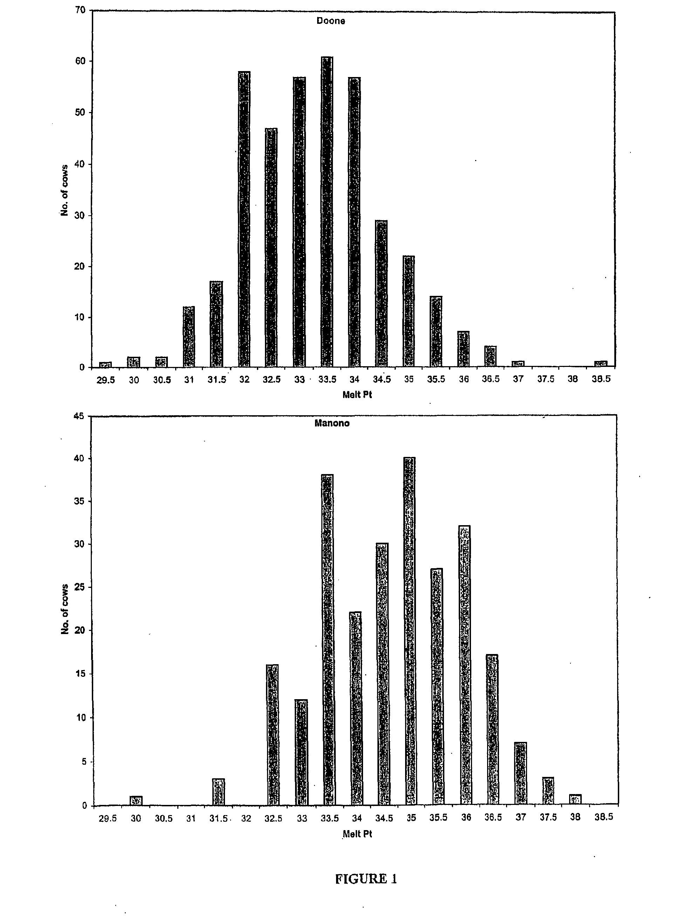

Figure 1 is a graph showing the results of the determination of Melting Point (hereinafter, "Melt Pt") of milk fat samples from individual cows from two large dairy herds located in the Doone and Manono regions of New Zealand. Melt Pt (in degrees Celsius) is plotted on the *X* axis, and the number of cows possessing a particular Melt Pt is indicated on the *Y* axis. The upper and lower panels depict results from the Doone and Manono herds, respectively.

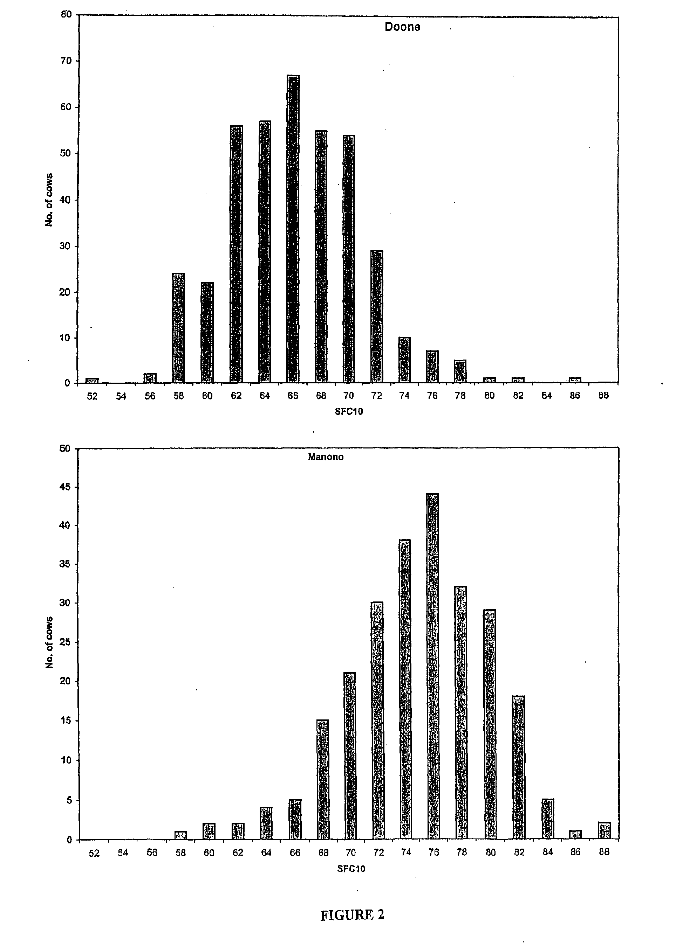

Figure 2 is a bar graph showing the results of the analysis of the "Saturated Fat Composition at 10 degrees Celsius" (hereinafter, "SFC 10") in milk samples from individual cows from the Doone and Manono herds. SFC 10 is plotted on the X axis, and the number of cows possessing a particular SFC 10 is indicated on the *Y* axis.

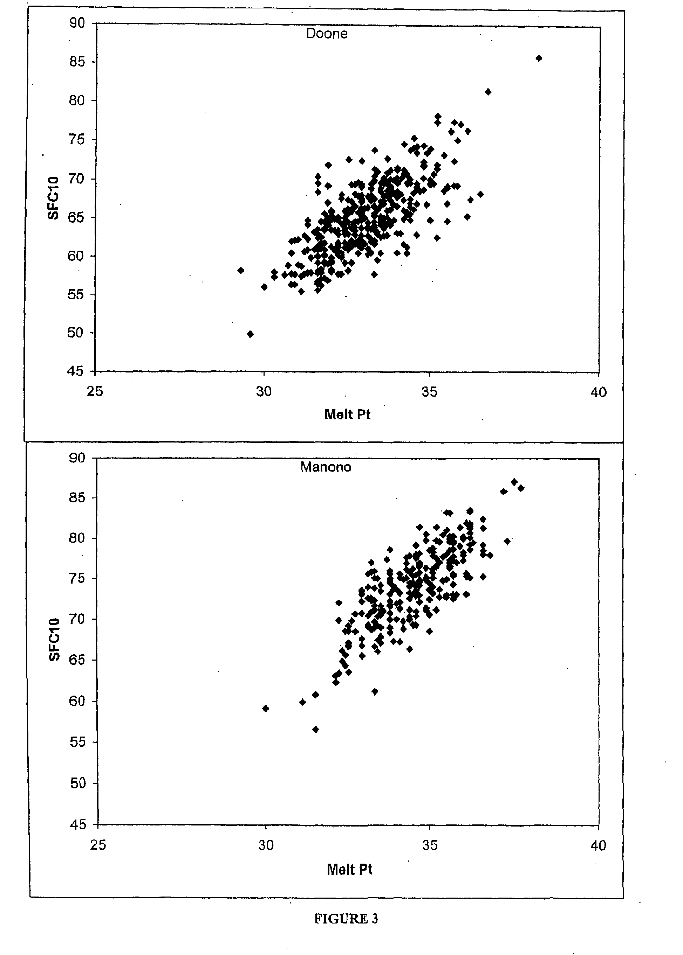

Figure 3 is a graph showing the relationship between the Melt Pt (plotted on the X axis) and the SFC10 (plotted on the *Y* axis), as determined for each milk sample from individual cows from the Doone and Manono herds. Each point represents milk fat from a single cow, and the upper and lower panels depict the results from the Doone and Manono herds, respectively.
   The correlation coefficients (or *r* values) describing the relationship between SFC10 and Melt Pt were *r* = 0.73 and *r* = 0.980 in the Doone and Manono herds, respectively.

### DETAILED DESCRIPTION

### A. Definitions

As used herein, "modified feed" refers to feed that has been processed to alter the fat composition of milk produced by animals consuming the modified feed. Modified feed includes feed that has been chemically processed or otherwise modified to allow passage through the rumen in a protected state, so that most of the hydrogenation of fatty acids takes place after the rumen. See, e.g., U.S. Patent Nos. 4,216,234; 5,670,191, 5,143,737; PCT Publication WO01/11978; Fogerty et al., 1980, Bull. Int. Dairy Fed.125:96; Storry et al., 1980, Bull. Int. Dairy Fed. 125:105-25. Modified feed also includes dietary supplements of unsaturated fatty acids (including calcium salts of long chain fatty acids, prilled or pelleted fats), full fat rape seed, heat treated/jet sploded oil seeds, or butyl soyamide esters administered to alter the fat composition of milk produced by animals consuming the modified feed. See, e.g. W. Christie, 1979, Prog. Lipid Res. 17:245; PCT Publication No. WO01/11978.

As used herein, "conventional diet" means a diet in which cows are not fed modified feed, as defined *supra.* A conventional diet includes pasture grazing, alfalfa hay, hay, corn, beans, grain, plant-based meal, plant-based haylage, plant-based silage, plant-based syrup; vitamins, minerals, and any mixture of any of these. "Pasture grazing" includes, but is not limited to, the consumption of the following grasses: timothy, cocksfoot, meadow fescue, tall fescue, reed canarygrass, and smooth broomgrass. Other forage includes, but is not limited to *L. perenne,* Lucerne and red clover. "Grains" include, but is not limited to, the following list of grains: oats, barley, maize and wheat.

As used herein, a "milk-producing cow" means a sexually mature female of genus *Bos* (generally two years of age, or older) who has had a calf and is producing milk or capable of producing milk. Generally, a cow will continue to produce milk if she produces a calf every year.

As used herein, the term "fatty acid" has the usual meaning in the art. Generally, "fatty acid" refers to long-chain organic acids having from 4-24 carbon atoms, a single carboxyl group and a long nonpolar hydrocarbon chain.

As used herein, "saturated fat" has the usual meaning in the art and refers to triacylglycerol(s) or triglyceride(s) in which the bound fatty acids are saturated.

As used herein, "saturated fatty acid," has the usual meaning in the art and means a fatty acid with only single bonds in the hydrocarbon chain. Typically, saturated fatty acids include the following fatty acids, which can be independently measured: lauric 12:0, myristic 14:0, palmitic 16:0, and stearic 18:0 fatty acids.

As used herein, the term "mono-unsaturated fatty acid (MUFA)" has the usual meaning in the art and refers to a fatty acid containing a single double-bond in the hydrocarbon chain of the molecule. MUFAs include oleic 18:1ₜₒₜₐₗ and oleic 18:1ₜᵣₐₙₛ, which can be independently measured.

As used herein the term "poly-unsaturated fatty acids (PUFA)" has the usual meaning in the art and refers to a fatty acid containing two or more double-bonds in the hydrocarbon chain of the molecule. PUFAs include linoleic 18:2 and linolenic 18:3, which can be independently measured.

As used herein, the term "fat composition" refers to the type and quantity of fatty acids found in the milk. The fat composition of milk can be described in terms of the amounts of total saturated fat, total mono-unsaturated fatty acids, total poly-unsaturated fatty acids. Unless otherwise specified, the quantity of a class of fatty acids contained in fat (triacylglycerol) is described as a percentage of all the fatty acid contained in triacylglycerol (% total fat) in the milk.

As used herein, the fat composition of a milk sample is "characteristic of" a milk product with a desirably modified fat or cholesterol (hereinafter, "MFAC milk") when the fat composition (i.e., amounts of specified fats and/or fatty acids) or cholesterol composition of the milk sample falls within the range for a MFAC milk described herein. For example, a milk sample containing 55% total saturated fat, 32% total MUFA, 10% total PUFA, 9% myristic 14:0, 18% palmitic 16:0, 26%, oleic 18:1ₜₒₜₐₗ, and 6% linoleic 18:2 has a fat composition characteristic of a MFAC milk with the following composition: total saturated fat [less than about 60%]; total MUFA [at least about 30%]; total PUFA [at least about 9%]. The milk sample also has a fat composition characteristic of a MFAC milk with the following composition: total saturated fat [less than about 60%]; myristic 14:0 [less than about 10%]; palmitic 16:0 [less than about 20%]; total MUFA [at least about 30%]; oleic 18:1ₜₒₜₐₗ [at least about 25%]; total PUFA [at least about 6%]; linoleic 18:2 [at least about 5%], lauric 12:0 [less than about 3.5%]; and linolenic 18:3 [at least about 1.5%]. A fat composition is not characteristic of MFAC milk when the fat composition is the same as that of the "control butter" in Table 2, *infra.*

As used herein, the term "pooled milk" refers to milk from a plurality of different cows that is combined (i.e., mixed together).

### B. Description

The present invention provides new methods, compositions and selected animal populations useful in the production of milk products with desirable properties. As described in Example 1, *infra,* human subjects consuming a diet containing a modified butter with the fat composition shown in Table 1 had significant decreases in cholesterol levels and both total and low density lipoprotein-C (LDL-C), with no significant change in high density lipoprotein-C (HDL-C), triglycerides (TG), or fasting glucose.

**TABLE 1**

| COMPOSITION OF MODIFIED BUTTER | |
|---|---|
| | (% composition) |
| total fat content (% w.w.) | 81.7 |
| moisture (% w.w.) | 15.4 |
| | (% total fat) |
| total saturated fat | 54.4 |
| lauric 12:0 | 2.7 |
| myristic 14:0 | 8.3 |
| palmitic 16:0 | 18.8 |
| stearic 18:0 | 13.4 |
| total MUFA | 32.0 |
| oleic 18:1ₜₒₜₐₗ | 30.0 |
| oleic 18:1*ₜᵣₐₙₛ* | 4.7 |
| total PUFA | 10.5 |
| linoleic 18:2 | 7.2 |
| linolenic 18:3 | 2.3 |
| | (mg/100g butter) |
| cholesterol | 191 |

These results demonstrated that a diet containing a modified dairy product (modified butter), in which a proportion of the saturated fats were replaced by fats containing monounsaturated (MUFA) and polyunsaturated (PUFA) fatty acids, had a striking effect on cholesterol and lipoprotein levels in humans. Two uncontrolled trials investigating the effect of a reduced saturates butter-fat on serum lipid profile and associated CVD risk factors (Noakes et al., 1996, Am. J. Clin. Nutr. 63:42; Tholstrup et al., 1998, Lipids 33:11) gave conflicting results, with only one of these trials suggesting an improvement in risk profile (Noakes et al., *supra*). In contrast, the present controlled study provides reliable evidence that consumption of dairy products with a modified milk fat composition favorably changes cholesterol and lipoprotein levels in humans.

Heretofore, dairy products with modified milk fat compositions generally have been produced by the addition of modified feed to the bovine diet. However, the use of modified feed is expensive. Moreover, in some circumstances, the processing used to modify the feed may be undesirable. For example, the use of formaldehyde-coated lipid supplement for ruminants producing milk or meat for human consumption may be unacceptable to consumers or regulatory agencies for aesthetic or safety reasons. Similarly, the feeding of oil supplements to cows may have the disadvantage of reducing milk production (see, e.g., U.S. Pat. No. 6,242,013).

The present inventors have, surprisingly, discovered that a proportion of cows fed a conventional diet produce milk with the desired characteristics of relatively low saturated fats and relatively high monounsaturated and polyunsaturated fatty acids. For example, 5-10% of New Zealand Friesian cattle fed a conventional diet produce milk with these desired characteristics (see Example 3). Based on this discovery, the invention provides compositions and methods useful for efficiently and economically producing milk products with a desirably modified fat or cholesterol composition (hereinafter, "MFAC milk").

The MFAC milk produced using the methods and bovine populations of the invention has the following composition (with all bracketed fat concentrations expressed as percentage of total fat): total saturated fat [less than about 60%]; total MUFA [at least about 30%]; total PUFA [at least about 9%].

Thus, in one embodiment, the MFAC milk has the following composition: total saturated fat [less than about 60%]; total MUFA [at least about 30%]; total PUFA [at least about 9%]. In a related embodiment, the MFAC milk has the following composition: total saturated fat [less than about 55%]; total MUFA [at least about 32%]; total PUFA [at least about 10%]. In another related embodiment, the MFAC milk has the following composition: total saturated fat [between about 50% and about 60%] total MUFA [between about 30% and about 40%]; total PUFA [between about 9% and about 11%].

In another related embodiment, the MFAC milk has the following composition: palmitic 16:0 [less than about 20%]. In a related embodiment, the MFAC milk has the following composition: palmitic 16:0 [less than about 19%]. In another related embodiment, the MFAC milk has the following composition: palmitic 16:0 [between about 15% and about 20%].

In another related embodiment, the MFAC milk has the following composition: total saturated fat [less than about 60%]; palmitic 16:0 [less than about 20%]; total MUFA [at least about 30%]; and total PUFA [at least about 6%]. In a related embodiment, the MFAC milk has the following composition: total saturated fat [less than about 55%]; palmitic 16:0 [less than about 19%]; total MUFA [at least about 32%]; and total PUFA [at least about 10%]. In another related embodiment, the MFAC milk has the following composition: total saturated fat [between about 50% and about 60%]; palmitic 16:0 [between about 15% and about 20%]; total MUFA [between about 30% and about 40%]; and total PUFA [between about 6% and about 12%].

In another related embodiment, the MFAC milk has the following composition: linoleic 18:2 [at least about 5%]. In a related embodiment, the MFAC milk has the following composition: linoleic 18:2 [at least about 7%]. In another related embodiment, the MFAC milk has the following composition: linoleic 18:2 [between about 5% and about 10%].

In another related embodiment, the MFAC milk has the following composition:
total saturated fat [less than about 60%]; total MUFA [at least about 30%]; total PUFA [at least about 6%]; and linoleic 18:2 [at least about 5%]. In a related embodiment, the MFAC milk has the following composition: total saturated fat [less than about 55%]; total MUFA [at least about 32%]; total PUFA [at least about 10%]; and linoleic 18:2 [at least about 7%]. In another related embodiment, the MFAC milk has the following composition: total saturated fat [between about 50% and about 60%]; total MUFA [between about 30% and about 40%]; total PUFA [between about 6% and about 12%]; and linoleic 18:2 [between about 5% and about 10%].

In another related embodiment, the MFAC milk has the following composition: total saturated fat [less than about 60%]; myristic 14:0 [less than about 10%]; palmitic 16:0 [less than about 20%]; total MUFA [at least about 30%]; oleic 18:1ₜₒₜₐₗ [at least about 25%]; total PUFA [at least about 6%]; linoleic 18:2 [at least about 5%]. In another related embodiment, the MFAC milk further comprises: lauric 12:0 [less than about 3.5%]; linolenic 18:3 [at least about 1.5%].

In another related embodiment, the MFAC milk has the following composition: total saturated fat [less than about 55%]; myristic 14:0 [less than about 8.4%]; palmitic 16:0 [less than about 19%]; total MUFA [at least about 32%]; oleic 18:1ₜₒₜₐₗ [at least about 30%]; total PUFA [at least about 10%]; linoleic 18:2 [at least about 7%]. In another related embodiment, the MFAC milk comprises: lauric 12:0 [less than about 3%]; linolenic 18:3 [at least about 2%].

In another related embodiment, the MFAC milk has the following composition: total saturated fat [between about 50% and about 60%]; myristic 14:0 [between about 6% and about 9%]; palmitic 16:0 [between about 15% and about 20%]; total MUFA [between about 30% and about 40%]; oleic 18:1ₜₒₜₐₗ [between about 25% and about 35%]; total PUFA [between about 6% and about 12%]; linoleic 18:2 [between about 5% and about 10%]. In another related embodiment, the MFAC milk further comprises: lauric 12:0 [between about 2% and about 3.5%]; linolenic 18:3 [between about 1.5% and about 3%].

Cholesterol levels in milk can also be measured. Typically, the cholesterol level in the MFAC milk is less than about 15 mg/100 g whole fluid milk, e.g., less than about 13 mg/100 g whole milk, for example.

### Populations of Dairy Cows Capable of Producing MFA C Milk

In one aspect, the invention provides a cow population where substantially all of the milk-producing cows in the population produce MFAC milk, as described *supra.* In particular, the milk-producing cows produce MFAC milk, or are capable of producing MFAC milk, when fed a conventional diet (e.g., a diet normally fed dairy cows in the country or region, not supplemented with modified fat, e.g. oil seeds). Thus, in one exemplary embodiment, the milk contains less than about 60% total saturated fat, at least about 30% mono-unsaturated fatty acids, and at least about 9% total poly-unsaturated fatty acids. In a second one exemplary embodiment, the milk contains less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2.

As used herein in this context, "substantially all of the milk-producing cows in the population" means at least about 50% of the milk-producing cows in the population, usually at least about 75%, more often about 90%, most often at least about 95%, or all of the milk-producing cows in the population. As described in detail *infra,* such a population can be made or maintained by selecting MFAC milk-producing cows from a heterogeneous population of dairy cows, through a breeding program, or by other means.

A cow population means a population of cows with at least about 10, more often at least about 50, and most often at least about 100 milk-producing cows. In some embodiments, the population contains at least about 150, at least about 200, at least about 500, or at least about 1000 milk producing cows.

Typically, a specified cow population, e.g., MFAC milk producing cows, is physically segregated from milk producing cows not in the population (i.e., milk producing cows that do not produce MFAC milk). Alternatively, individuals in the a specified population can be physically comingled with other cows, but identified as MFAC milk producing individuals using identification tags, implantable micro-chips, or other identification methods known in the dairy art by which characteristics of individual cows are recorded. For convenience, these identified cows are referred to as "informationally segregated cows." One purpose of the physical (usual) or informational segregation is to provide the practitioner an efficient, convenient, and economical way to keep the milk of cows producing MFAC milk separated from that of other cows is retained. It will be apparent to those of ordinarily skilled practitioner that the population of milk producing cows need not necessarily be segregated from non-milk producing cows, e.g., juveniles and males.

The present invention contemplates that the cow population can include any of a variety of dairy cow breeds (or even a mixture of breeds). Suitable cow breeds include Friesian, Guernsey, Holstein, Ayreshire, Jersey, Brown Swiss, Milking Shorthorn; Simmental, Girolando, Sahiwal and other *Bos indicus* milking breeds; as well as other breeds known in the art.

It will be appreciated that in certain circumstances it may be desirable to feed modified feed, as described above, to a cow (or population of cows) of the invention, i.e., one capable of producing MFAC milk when fed a conventional diet. For example, it is possible that the feeding of the modified feed to cows capable of producing MFAC milk when fed a conventional diet would further reduce the levels of saturated fats in the milk produced by the cows.

### Identification of Dairy Cows Capable of Producing MFA C Milk

To determine whether an individual cow produces MFAC milk, the fat composition and/or cholesterol composition of the milk from the individual is measured. Any suitable method for analysis of milk fats is suitable. Generally, a milk sample is obtained from an individual cow. Means for obtaining a representative milk sample are well known in the art. The milk sample may be frozen, or may be subjected to further analysis without freezing. The fat composition of the milk sample is measured using methods well known in the art as described *infra,* and the type and quantity of fatty acids and/or cholesterol present in the milk sample can be recorded. Most often, the individual cow is fed a conventional diet, e.g., for at least about three days, and preferably at least about five days prior to the collection of the milk sample.

Methods for determining the type and quantity of fats and fatty acids are known and are described in, e.g., Cook et al., 1972, J. Dairy Res. 39:211; Noakes et al., 1996, Am. J. Clin. Nutr. 63:42; U.S. Pat. No. 6,242,013. Typically, total fat is determined by extraction from a tissue or fluid, such as milk (or butter made from the milk), by mixing or homogenizing with a suitable solvent such as chloroform, chloroform/ethanol or chloroform/isopropanol, diethyl ether, or petroleum ether, or mixtures such as NH₄OH/ethanol/diethyl ether/petroleum ether (Walstra & Mulder, 1964, Neth. Milk Dairy J 18: 237), followed by gravimetric analysis. Alternate volumetric methods employ H₂SO₄ to liberate fat, which is then measured. See, e.g., Ling, 1956, A Textbook of Dairy Chemistry, 3rd ed. Vol. 2, Practical, Chapman Hall, London; Horwitz, ed., 1980, Official Methods of Analysis, 13th ed., Association of Official Analytical Chemists, Washington, D.C. Rapid determination of the amount of fat in milk can be done by measurement of the absorption of infrared radiation at 3.4 or 5.7 µm (e.g., Horwitz, *supra;* Goulden 1964, J. Dairy Res.; 31:273).

The fatty acid type and quantity of fat and fatty acids in the extracted fats may be further characterized by chemical cleavage and characterization of fatty acids using, for example, gas-liquid chromatography (hereinafter, "GLC") (e.g., James & Martin, 1956, Biochem. J. 63:144; Jensen et al., 1962, J. Dairy Sci. 45:329; Jensen et al., 1967, J. Dairy Sci. 50:19), in which fatty acids are determined by separation of mixtures of volatile fatty acid derivatives, for example methyl derivatives formed by transesterification with sodium methoxide (Christopherson & Glass, 1968, J. Dairy Sci. 52:1289). Alternatively, fatty acids may be esterified using sodium butoxide or H₂SO₄ and boron trifluoride catalyzed butyrolysis (Iverson & Sheppard, 1977, J Assn Off Anal Chem 60:284), enabling determination as butyl esters (e.g., Christopher & Glass, *supra;* Parodi, 1970, Aust. J. Dairy Technol. 25:200). Alternatively, milk fatty acids may be determined by GLC-mass spectrometry following argentation thin layer chromatography (hereinafter, "TLC") (e.g., Strocchi & Holman, 1971, Riv. Ital. Sostanze Grasse 48:617), or by high resolution open-tubular GLC (e.g., Ackman et al., 1972, Lipids 7:683). The total amounts of conjugated fatty acids present in milk fat extracts have been determined by ultraviolet spectrophotometry (see, e.g., Smith et al., 1978, J. Am. Oil Chem. Soc. 55:257). Milk lipid classes from extracts can also be separated and classified by TLC (see, e.g., Smith et al, *supra*).

Free fatty acids may be analyzed and quantified in plasma by GLC, following extraction, for example as described in Dol, 1956, J. Clin. Invest. 35:150; Turnell et al., 1980, Clin. Chem. 26:1879. Serum triglycerides may be measured following hydrolysis by a mixture of lipase and esterase, with determination of glycerol by kinetic fixed-time analysis additionally using glycerol kinase, pyruvate kinase, and lactate dehydrogenase (see, e.g., Ziegenhorn, 1975, Clin. Chem. 2:1627; Klotzsch & McNamara, 1990, Clin. Chem. 36:1605).

The cholesterol composition of the milk may be quantified using GLC-mass spectrometry of trimethylsilyl esters (e.g., Mincione et al., 1977, Milchwissensch 132:107), or by GLC (see, e.g., Parodi, 1973, Aust J Dairy Sci 28:135). See also, e.g., LaCroix et al., 1973, J. Am Diet Assn 62:275.

To determine whether the individual cow produces MFAC milk, the fat composition of the individual cow being tested is compared with a reference fat composition, e.g., the fat composition of a MFAC milk as described hereinabove.

The fat composition of milk may also be determined by making butter from the milk and measuring the fat composition of the butter produced. The fat compositions of milk and butter made from the milk are essentially identical (see, e.g., Jensen, ed., 1995, Handbook of Milk Composition, Academic Press, New York, NY).

### Methods of Generating a Population of Dairy Cows Capable of Producing MFAC Milk by Selection

In one aspect, the invention provides a method of generating a cow population described *supra,* i.e., where substantially all of the milk-producing cows produce MFAC milk. In one embodiment, the method involves obtaining a milk sample from several (e.g., at least 3, but typically more, e.g., at least about 10) individual cows and determining whether the fat composition of the milk sample is characteristic of a MFAC milk as described herein (e.g., comprising less than about 60% total saturated fat, at least about 30% mono-unsaturated fatty acids, and at least about 9% total poly-unsaturated fatty acids). According to the method, individual cows that produce milk with a desired MFAC composition are physically or informationally segregated from non-MFAC cows. Any number of cows can be screened and segregated to generate a population of MFAC milk producing cows.

### Methods of Generating a Population of Dairy Cows Capable of Producing MFAC Milk by Breeding

In one aspect, the invention provides a method of generating a progeny cow or cow population, where the cow or substantially all of the milk-producing cows in the population, produce MFAC milk. In one embodiment, the method involves identifying at least one cow that, when fed a conventional diet, produces milk with a fat composition characteristic of a MFAC milk, breeding the cow to produce progeny; and selecting progeny that produce milk with a milk fat composition characteristic of a MFAC milk. In one embodiment, the method involves obtaining a milk sample of an individual cow, comparing the fat composition of the milk sample to a reference milk fat composition characteristic of MFAC milk; breeding cows that produce milk with a fat composition characteristic of MFAC milk to generate progeny cows producing milk of the desired lipid profile. Usually, progeny with the desirable characteristics (i.e., the ability to produce MFAC milk) are segregated from other cows, thereby producing a population of cows where substantially all of the milk-producing cows in the population produce MFAC milk.

Standard cattle breeding methods useful in the practice of the invention are well known. See, e.g., D. C. Dalton "An Introduction to Practical Animal Breeding," 2nd ed., Collins, London, England; and D.S. Falconer, "Introduction to Quantitative Genetics," Agricultural Research Council's Unit of Animal Genetics, University of Edinburgh, Ronald Press Co., New York, N.Y., in particular chapters 11 and 12. Daughters of sires of high genetic merit, whose semen is widely used, are tested for their ability to produce MFAC milk. As the heritability of this trait appears to be high, sires that generate many daughters that produce MFAC milk can be used to cross with MFAC-producing cows. In this way, the proportion of MFAC milk-producing cows in a given dairy herd is increased.

### A Method of Identifying an Individual Cow Capable of Producing MFAC Milk

In another aspect, the invention provides a method of identifying an individual cow that produces MFAC milk in the absence of the need to administer a modified feed diet. In an embodiment, the method involves (a) obtaining a milk sample produced by an individual cow that has been fed a conventional diet for at least about three days, preferably at least about five days, sometimes at least about 30 days, prior to the time the sample is obtained; (b) determining whether the fat composition of the milk sample is characteristic of a MFAC milk; and (c) identifying an individual cow that produced a milk sample with a fat composition characteristic of a MFAC milk as a milk-producing cow that produces MFAC milk. The fat composition of the milk sample is measured using routine methods, such as those described herein, and compared to a reference value characteristic of MFAC milk. Reference profiles are MFAC fat and/or cholesterol amounts as described herein. For example, a first reference profile is "less than about 60% total saturated fat, at least about 30% mono-unsaturated fatty acids, and at least about 9% total poly-unsaturated fatty acids." A second reference profile is "less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2." Other reference profiles useful in the practice of the invention will be apparent from the present disclosure. In a one embodiment, the identification method is applied to a number of individual cows (e.g., at least 5, at least 10, at least 25 or more) to identify a plurality of cows that produce, or are capable of producing, MFAC milk.

Typically, the identified cows are segregated (physically or informationally) to produce a cow population for production of MFAC milk.

### Pooled Milk Compositions Containing Milk from Cows Producing MFAC Milk

In various aspects, the present invention provides populations of cows where substantially all of the milk-producing cows in the population produce MFAC milk, methods of generating such populations (e.g., by selection, breeding, and segregation), and methods of identifying individual cows that produce MFAC milk. In related aspects, the invention provides pooled milk from a plurality of individual milk producing cows that are capable of producing MFAC milk (e.g., milk containing less than about 60% total saturated fat, at least about 30% mono-unsaturated fatty acids, and at least about 9% total poly-unsaturated fatty acids, such as milk containing less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2) when fed a conventional diet. As used herein, a plurality of cows means at least two, at least three, at least 5, at least about 10, at least 50, at least about 100, or at least about 200 cows.

It is contemplated that, in some embodiments, the pooled milk composition does not contain (or contains only insignificant amounts) of milk from cows other than cows that produce milk-producing cows in the population produce MFAC. Usually at least about 50% of the milk in the pooled milk composition is from cows capable of producing MFAC-milk when fed a conventional diet, more often at least about 75%, more often at least about 90%, more often at least about 95%. However, the pooled milk of the invention can be combined with milk from different sources, if desired. It will be recognized that pooled milk from a plurality of cows that produce MFAC milk will have the composition of MFAC milk (e.g., milk containing less than about 60% total saturated fat, at least about 30% mono-unsaturated fatty acids, and at least about 9% total poly-unsaturated fatty acids) when measured without further processing to remove or add fats or fatty acids. Further, although the pooled milk can in principle be combined with milk from conventional cows, in general the final milk product will have the composition of MFAC milk (e.g., milk containing less than about 60% total saturated fat, at least about 30% mono-unsaturated fatty acids, and at least about 9% total poly-unsaturated fatty acids) when measured without processing to remove or add fats or fatty acids.

In another aspect, the invention provides a pooled milk composition comprising milk from a plurality of individual cows fed conventional diet(s), where the pooled milk composition possesses a fat composition characteristic of the fat composition of a MFAC milk. The plurality of individual cows can include individual cows capable of producing MFAC milk, individual cows not capable of producing MFAC milk, or both.

The invention also provides products produced from, or made using, the pooled milk *supra,* e.g., dried, condensed, and skim milk, cream, ice cream, chocolate, butter, cheese, yogurt, or infant formula. In one embodiment, a "product" means a food that contains fat obtained from MFAC milk obtained from cows segregated according to the methods of the invention.

*Method of Identifying a Cow with a Genotype Indicative of Production of MFAC Milk* In another aspect, the invention provides a method of genetic evaluation of cattle by assaying for the presence of at least one genetic marker associated with the trait of production of MFAC milk. The ability to identify such a genetic marker permits marker-assisted breeding, in which, for example, young bulls can be identified by genetic testing as having marker(s) for desirable traits, and the necessity for progeny testing can be avoided. Similarly, females identified as having such marker(s) can be super-ovulated, and resulting eggs fertilized *in vitro* and implanted in other females allowing for the use of the superior genetics of the female (or male) without having to wait for her to give birth to one calf at a time. Further, cows identified as having favorable markers can be targeted for a desired feeding regimen.

The method involves (1) identifying a cow that produces, or is capable of producing MFAC milk, as described *supra,* (2) obtaining a nucleic acid sample of the cow, and (3) assaying the sample for the presence of a polymorphism(s) associated with production of MFAC milk. In a related embodiment, the method involves (1) identifying a cow that produces, or is capable of producing MFAC milk, as described *supra,* (2) obtaining a nucleic acid sample of the cow, and (3) assaying the sample for the presence of a polymorphism in a milk metabolism-related gene or a milk composition-related gene in the sample. As used herein, a "milk metabolism-related gene" means a gene known or determined to be associated with production of milk fat, e.g., stearoyl CoA desaturase, lisophosphatidic acid acyl transferase (LPAT), fatty acid synthetase, glycerol-3-phosphate acyltransferase, thioesterase I and II, etc. As used herein, a "milk composition-related gene" is a gene whose expression has been implicated in the production of milk, production of milk of a particular composition, and/or the regulation of milk fat composition, e.g., genes involved in fatty acid synthesis and metabolism, which genes are well known in the art.

Standard methodology for identifying polymorphism(s) associated with a particular phenotype (the capacity to produce MFAC milk) is known. In general, the methodology involves obtaining nucleic acids from individual cows of MFAC and non-MFAC phenotypes, and assaying nucleic acids for polymorphism(s) are associated with the presence or absence of the production of MFAC milk. Methods for carrying out these assays generally include extraction of DNA, digestion with restriction enzymes, and separation of the resulting fragments, hybridization to radio-labeled probe(s), e.g., as in U.S. Pat. Nos. 5,614,364 and 6,242,191; Sambrook et al., Molecular Cloning- A Laboratory Manual, 2nd and 3rd editions., Cold Spring Harbor Press, Cold Spring Harbor, N.Y. Use of the polymerase chain reaction (PCR) to amplify the relevant gene fragment for further analysis, is also included in standard methodology. Analysis of polymorphisms is described in, e.g. U.S. Patent Nos. 5,614,364; 5,939,264; 6,242,191; and in, for example, D. C. Dalton "An Introduction to Practical Animal Breeding," 2nd ed., Collins, London, England, and D.S. Falconer, "Introduction to Quantitative Genetics," Agricultural Research Council's Unit of Animal Genetics, University of Edinburgh, Ronald Press Co., New York, N.Y. Briefly, presence or absence of a genetic marker in a sample of cows is compared with the milk composition phenotype. Statistical methods are applied to estimate the significance of the association of the marker and the phenotype. Useful statistical methods are known, e.g., U.S. Pat. No. 5,614,364; Wiggam et al., J. Dairy Sci. (Supp. 2) 71:54, and the *Analysis of Variance (ANOVA)* program of the Statistical Analysis Software (SAS) program from the SAS Institute Inc., Cary, N.C. Individual animals are screened for a genotype indicative of production of MFAC milk using standard methods. See, e.g. U.S. Pat. Nos. 5,614,364; 5,939,264; 6,242,191. The animal subjected to be screening may be a cow, a male or female calf, or a bull. The animal may be juvenile, sexually mature, fertile, infertile, or past the period of fertility.

### C. Examples

The following Examples are provided to illustrate, but not limit, the invention.

### EXAMPLE 1

### STUDY OF THE BLOOD-LIPID LOWERING POTENTIAL OF A NATURAL BUTTERFAT CONTAINING INCREASED UNSATURATED FATTY ACIDS

This example describes a study performed to determine the efficacy of lowering blood lipid composition by using a natural butterfat containing increased unsaturated fatty acids.

We found significant decreases in both total and LDL-C during the feeding of modified butter, but no significant changes in HDL-C, TG, or fasting glucose. We conclude that clinically significant improvement in cardiovascular risk can be achieved by moderate changes in dietary fatty acid profile achieved through a common and well accepted food source, butterfat.

### Subjects

Twenty healthy, male volunteers were recruited into the study following advertisement for interested participants. All were of normal body weight (Body mass index = 18-25 kg/m²) and, following screening were shown to have normal blood lipids, liver function, thyroid function (as assessed by thyroxin and TSH), fasting plasma glucose and insulin concentrations, and blood pressure. None had a known history of cardiovascular disease or diabetes, nor were currently or previously treated for hypertension or any known metabolic disorder. All volunteers provided written informed consent. Ethical approval for the study was obtained from the University of Auckland and from the Auckland North Health Authority Ethics Committees.

### Protocol

This study was a double blind, randomized, controlled dietary intervention in which compliance was ensured by provision and monitoring of consumption of all foods and beverages. All subjects were randomly assigned to initially enter either the treatment or control arm of the trial, and were required to be resident at the University of Auckland Human Nutrition & Metabolic Unit (hereinafter, "Metabolic Unit") throughout both dietary intervention periods. Each of the two intervention periods lasted for 21 days, during which blood and urine samples were regularly collected. Fasted blood samples were collected by venipuncture on the morning of days 0 and 1 (pre-intervention baseline), 7, 14, 21 and 22. 24-h urine samples to assess dietary compliance by nitrogen balance were collected on days 10 and 20 on both arms of the intervention. Body weight was measured daily whilst subjects were fasted and after voiding of the bladder. Blood samples were analyzed for total cholesterol and fractions, triacylglycerol, apoA, apoB, non-esterified fatty acids (NEFA), fasting glucose, fasting insulin, and hemostatic factors fibrinogen and factor VII.

### Butterfat composition

The composition of the two butterfats used in this trial is shown in Table 2. In the modified butter, a proportion of the saturated fats were replaced by fats containing monounsaturated (MUFA) and polyunsaturated (PUFA) fatty acids. Saturated fat was decreased from 70.5% total fat in the control butter to 54.4% in the modified butter. Concomitantly, total MUFA was increased from 22.1% to 32.0% total fat, and total PUFA from 3.0% to 10.5%. The major MUFA increase occurred in the oleic acid (18:1ₜₒₜₐₗ) fraction, which was raised from 18.6% total fat (control) to 30.0% (modified). In the PUFA, the major increment occurred in the linoleic acid (18:2) fraction, which was increased from 1.2% total fat (control) to 7.2%. In addition, the cholesterol content of the modified butter was slightly lower (191 mg/100 g butter) compared with that of the control butter (222 mg/100 g). The fatty acid composition of the unmodified (Jensen et al, 1962, J. Dairy Sci. 45:329; Hansen and Shorland, 1952, Biochem. J. 52:207) and modified butters (Fogerty and Johnson, 1980, Bull. Int. Dairy Fed. 125:96; Storry et al, 1980, Bull. Int. Dairy Fed. 125:105) determined in this study is consistent with those reported by others.

**Table 2**

| Composition of Control and Modified Butter Fats | | | |
|---|---|---|---|
| % composition | Control butter | Modified butter | delta |
| | | | |
| total fat content (% w.w.) | 85.2 | 81.7 | -3.5 |
| moisture (% w.w.) | 12.4 | 15.4 | +3.0 |
| total saturated fat (% fat) | 70.5 | 54.4 | -16.1 |
| lauric 12:0 | 3.8 | 2.7 | -1.1 |
| myristic 14:0 | 12.0 | 8.3 | -3.7 |
| palmitic 16:0 | 31.5 | 18.8 | -12.7 |
| stearic 18:0 | 10.1 | 13.4 | +3.3 |
| total MUFA (% fat) | 22.1 | 32.0 | +9.9 |
| oleic 18:1ₜₒₜₐₗ | 18.6 | 30.0 | +11.4 |
| oleic 18:1*ₜᵣₐₙₛ* | 4.3 | 4.7 | +0.4 |
| total PUFA (% fat) | 3.0 | 10.5 | +7.5 |
| linoleic 18:2 | 1.2 | 7.2 | +6.0 |
| linolenic 18:3 | 0.8 | 2.3 | +1.5 |
| cholesterol mg/100g butter | 222 | 191 | -31 |

The modified high MUFA butterfat was manufactured for this trial using cow feeding methods. Lactating dairy cows were fed a diet enriched with unsaturated fatty acids, protected from saturation in the rumen by an encapsulating coat, to promote increases in the MUFA and PUFA content and to decrease concomitantly the saturated fatty acid content of the milk from which the butterfat was derived (Cook et al, 1972, J. Dairy Res. 39:211; Fogerty and Johnson, 1980, *supra;* Storry et al, 1980, *supra;* Noakes et al., 1996, *supra*)*.* The only dairy fat product given to subjects in this intervention was the control and modified dairy butter. No cheese, yogurt, spreads or dairy-derived lipid products of any kind were included in the background diet.

### Diet

Background diet was designed to be identical on both arms of the intervention to ensure that the only difference between the diets was the fatty acid profile driven by the composition of the control and modified butterfats. The total dietary intake, including the butterfat supplement, for all subjects is shown in Table 3. The diet was controlled for total fat and cholesterol, total carbohydrate (CHO) and fiber, total protein and protein fractions, and micronutrients including Na, K, and Ca. To ensure both treatments were identical all food ingredients were weighed to the nearest gram during diet preparation. The energy and macronutrient content of the diet was initially calculated using the dietary program 'Diet 1' (Crop & Food Research, Palmerston North, New Zealand) and then verified by direct chemical analyses of duplicate diet samples. The duplicate diet methodology was such that on 12 occasions during the intervention a duplicate 4 day diet from a single subject was collected, homogenized and an aliquot frozen for later chemical analysis. This enabled the absolute composition of the diet to be verified and also demonstrated that there were no significant trends caused by seasonal variability in food products included in the diet. Butterfat provided half of the total fat in the diet (= 20 percentage nutrient energy, hereinafter "en%"), and hence was scaled to total energy intake and body weight for each individual.

**Table 3**

| Composition of the Diets Including the Butter Supplements as Measured by Direct Chemical Analysis (Mean ± S.D.)* | | | |
|---|---|---|---|
| | Control butter | Modified butter | delta |
| | | | |
| energy intake, EI (range, MJ/d) | 10.5 - 15.5 | 10.5 - 16.0 | |
| EI (mean, MJ/d)⁺ | 13.1 t 0 | 13.2 ± 0.2 | +0.1 |
| CHO,% of energy⁺ | 47 ±0.6 | 48 ± 0.6 | +1 |
| Protein,% of energy⁺ | 13 ±0.7 | 13 ± 0.7 | 0 |
| Fat,% of energy⁺ | 40 ±0.8 | 39 ±0.8 | -1 |
| Total SFA (calculated, en%) | 20 ±0.3 | 15 ± 0.3 | -5 |
| SFA profile (mg/g) | | | |
| C10:0 | 3.1 | 2.6 | -0.5 |
| C12:0 | 12.9 | 14.4 | +1.5 |
| C14:0 | 16.1 | 8.8 | -7.3 |
| C16:0 | 37.4 | 26.6 | -10.8 |
| C18:0 | 12.7 | 16.8 | +4.1 |
| Total MUFA (calculated, en%) | 6 ± 0.2 | 8 ± 0.1 | +2 |
| MUFA profile (mg/g) | | | |
| C16:1 | 3.2 | 2.3 | -0.9 |
| C18:1 | 31.6 | 44.0 | +12.4 |
| Total PUFA (calculated, en%) | 14 ±0.1 | 16 ± 0.2 | +2 |
| PUFA profile (mg/g) | | | |
| C18:2 | 34.1 | 44.8 | +10.7 |
| C18:3 | 2.7 | 3.6 | +0.9 |
| Cholesterol (mg/100g)⁺ | 45.9 | 40.4 | -5.5 |

| | | | |
|---|---|---|---|
| *Results are means for 6 duplicate homogenized portions analyzed from each of Control and Modified diets. The major effects were a reduction in C14:0, C16:0 and an increase in C18:0, C18:1, C18:2 in the Modified diet. ⁺No significant difference between treatments (P>0.05). Minor components in the fatty acid profiles are not shown. SFA, saturated fatty acids; MUFA, monounsaturated fatty acids; PUFA, polyunsaturated fatty acids. | | | |

Subjects were fed to energy balance, based on a multiple of predicted basal metabolic rate (BMR; Schofield et al., 1985, Hum. Nutr. Clin. Nutr. 39C (Suppl.) 1) and diets were altered on a daily basis to maintain a constant body weight during each intervention period. A combination of change in body weight, reported activity and hunger levels were used to assess total daily energy requirements. A 4 day dietary rotation was used during the study such that every 5^{th} day the entire diet was repeated. Subjects were provided with breakfast, lunch, dinner and between-meal snacks. Breakfast and dinner were eaten under supervision at the Metabolic Unit, whilst lunch and snacks were packed and volunteers were able to take them to college or their place as work as required. Decaffeinated, sugar-free beverages and decaffeinated tea and coffee were freely available. Subjects were required to eat only and all of the foods provided and no others. Alcohol was prohibited throughout the intervention. The subjects were self selected and highly motivated. Independent dietary compliance was assessed from 24-h urinary nitrogen balance data, where urinary losses of nitrogen were directly compared with dietary protein intake (where g protein = 6.25 x g Nitrogen).

### Statistical analyses

*T*-test analyses were used to identify any differences in dietary energy or macronutrient composition between the 'modified' and 'control' diets as eaten by the subjects (background diet + butter-fat supplement). All anthropometric and metabolic variables including body weight, total-, LDL- & HDL-C, TG, apoA, apoB, fibrinogen and factor VII were analyzed for between-diet effects with time and subject interactions, using split-plot-in-time repeated measure single factor ANOVA. These data were also analyzed for longitudinal changes between baseline and the end of the intervention on each treatment separately from repeat measures ANOVA, assessing the change in slope over the entire 21 days of the intervention. All baseline data was calculated as the mean (± s.e.m.) of the 2 pre-intervention blood samples collected on days 0 & 1. The repeat measure on each individual was performed to increase the accuracy of baseline. All biochemical assays were analyzed in triplicate and presented as a mean ± s.e.m. Statistical significance was based on 95% confidence limits (P<0.05).

### Results

In this intervention, the cow feeding regimen was able to achieve an ∼16% decrease of saturated fatty acids within the butterfat, replaced by ∼9% and ∼8% increases in MUFA and PUFA, respectively. The major reductions were in palmitic (C16:0, -12.7%) and myristic (C12:0, -3.7%) acids. Oleic acid (C18:1) increased by 11.4%, linoleic (C18:2) by 6.0% and linolenic by 1.5%. The macronutrient composition of the total diet consumed, including the butter supplement, was on average 39% of total energy derived from fat, 48 en% carbohydrate and 13 en% protein (Table 3). There was no significant difference between total energy or macronutrient composition between treatments (P>0.05). As intended in the study design, the considerable differences in composition between the two butterfats resulted in a significant difference in fatty acid profile between the two treatment diets. There was also a difference in dietary cholesterol between treatments, reflecting the 14% decrease in the modified butterfat relative to the control product.

Subject motivation and compliance were maximized in this residential study by provision of all foods and beverages throughout both intervention periods. Compliance for each subject was assessed by 24-h Nitrogen balance on 4 occasions during the trial (results not shown). Body weight and metabolic outcomes pre- and post intervention are shown in Table 4. There was no significant difference at baseline between the control and modified butters for any of the parameters measured (P>0.05). There was no significant difference in the average body weight of the subjects during the 3 weeks of modified or control butter feeding, nor was there a significant increase or decrease during either intervention period which would have influenced lipid profile (P>0.05). Body weight was successfully maintained within limits of ± 2 kg of the baseline weight on both arms of the intervention.

Table 4 shows the total, LDL- and HDL-C before and after both the control and modified butter interventions. There was a significant treatment effect in this intervention, such that the concentrations of both the total and LDL-C decreased when subjects were fed the diet containing fat derived from the modified product. Both total- (P<0.05) and LDL-C (P<0.01) were significantly decreased when subjects were fed the modified butter-containing diet when compared with the control diet; this change was sustained throughout the 3-week intervention. In addition to the between treatment effect there was also a significant decrease relative to baseline within both treatments. Total serum cholesterol decreased by -0.36 mmol/L (P<0.001) between baseline and day 22 on the modified butter, and by -0.24 mmol/L (P<0.01) on the control butter. When calculated as percentage change from baseline, by day 22 total cholesterol had decreased by -7.9% and -5.3% respectively. The modified butter also decreased LDL-C between baseline and the end of the intervention by -0.28 mmol/L (-9.5%, P<0.01) and remained virtually unchanged on the control butter (-0.07 mmol/L; -2.4%, P>0.05). There was no significant difference in HDL-C (P>0.05) between butter treatments during the 3 week intervention, nor was there a significant change between baseline and end of the intervention on the modified butter treatment (P>0.05). There was however a longitudinal decrease in HDL-C on the control treatment (P<0.05). Circulating triglyceride levels were also unaffected when compared across treatments (P>0.05, Table 4), but both modified (P<0.01) and control (P<0.05) butter arms of the intervention reduced triglyceride over the 3 weeks. There was a trend for total-C/HDL-C and LDL-C/HDL-C ratios to both decrease on the modified butter (total-C/HDL-C, 8=-0.18; LDL-C/HDL-C, 5=-0.15), but these effects did not reach statistical significance (P>0.05). There was no significant treatment effect for either of the clotting factors measured, fibrinogen or factor VII (P>0.05), nor did either variable significantly change relative to baseline during intervention (Table 4). There were no significant between treatment effects on apoA, apoB, NEFA or fasting blood glucose (P>0.05).

**Table 4 Effect of Control and Modified Butter Treatments on Body Weight and Metabolic Risk Factors of Twenty Healthy Adult Men**

| Variable | Control butter | | Modified Butter | |
|---|---|---|---|---|
| | Pre-treat. | Post-treat. | Pre-treat. | Post-treat. |
| body weight (kg) | 68.7 ± 6.1 | 68.4 ± 6.0 | 69.4 ± 6.2 | 69.3 ± 5.9 |
| total cholesterol (mmol/L) | 4.54 ± 0.5 | 4.31 ± 0.6 | 4.58 ± 0.7 | 4.22 ± 0.7* |
| LDL-cholesterol (mmol/L) | 2.92 ± 0.5 | 2.85 ± 0.6 | 2.98 ± 0.6 | 2.70 ± 0.5** |
| HDL-cholesterol (mmol/L) | 1.24 ± 0.3 | 1.16 ± 0.3 | 1.22 ± 0.3 | 1.19 ± 0.3 |
| triglyceride (mmol/L) | 0.84 ± 0.4 | 0.69 ± 0.3 | 0.85 ± 0.3 | 0.74 ± 0.2 |
| apolipoprotein A (g/L) | 1.67 ± 0.2 | 1.62 ± 0.2 | 1.66 ± 0.2 | 1.61 ± 0.2 |
| apolipoprotein B (g/L) | 0.81 ± 0.1 | 0.75 ± 0.1 | 0.82 ± 0.2 | 0.74 ± 0.1 |
| fibrinogen (g/L) | 2.78 ± 0.4 | 3.02 ± 0.8 | 2.95 ± 0.9 | 2.74 ± 0.7 |
| factor VII (U/L) | 937 ± 218 | 915 ± 265 | 873 ± 252 | 853 ± 29 |

| | | | | |
|---|---|---|---|---|
| Values are means ± S.D. There was no significant difference between control and modified butter populations pre-treatment for any measured variable. Pre-intervention was calculated as the mean of values corresponding to day-0 and day-1; post-treatment, day-22. Significant effect of treatment, ANOVA: *P<0.05, **P<0.01 | | | | |

### EXAMPLE 2

### ANALYSIS OF MILK

This example shows a method for analysis of the fat composition of a milk sample.

### Extraction of lipids and separation offatty acids

Total lipids are extracted from a milk sample by a modified version of the method of Bligh and Dyer [Gorski J, Nawrocki A & Murthy M. (1998), Characterization of free and glyceride-esterified long chain fatty acids in different skeletal muscle types of the rat. Molecular and Cellular Biolody 178:113-118 & Kates M, (1986), Techniques of Lipidology in Laboratory Techniques in Biochemistry & Molecular Biology, Vol 3: Pt2. Eds Burdon RH & van Knippenberg PH, Elsevier, Amsterdam, pp 100-111; Bligh EG & Dyer W J, (1959), A rapid method of total lipid extraction and purification. Can J Biochem Physiol 37:911-917. Kaluzny MA, Duncan LA, Merritt MV & Epps DE. (1985) Rapid sparation of lipid classes in high yield and purity using bonded phase columns. J Lipid Research 26:135-140. Prasa MR, Jones RM, Young HS, Kaplinsky LB & Das DK. (1988) Analysis of tissue free fatty acids isolated by aminpropyl bonded-phase columns. J Chromatography 428: 221-228]. 2 volumes (hereinafter "v") methanol containing 0.005% butylated hydroxy toluene (BHT) and 1v chloroform is added to 1v of milk. The mixture is vortexed at maximum speed for 2 minutes and centrifuged at 2,500 g for 4 minutes. The supernatant is recovered and the pellet reextracted with 2v methanol, 1v chloroform and 0.8v of 0.2 N HCl. The residue is vortexed for 2 minutes and centrifuged at 3,500 g for 3 minutes. After centrifugation the combined supernatants are diluted with 2v each of milli Q water and chloroform and the phases separated by centrifugation at 3,500 g for 4 minutes. The lower chloroform phase is recovered, neutralized by dropwise addition of 0.2 N methanolic NH₄OH and evaporated down in a stream of N₂. The samples are stored at -80°C until required.

Aminopropyl phase (250 mg) is packed into 16 ml teflon columns with teflon frits placed at the top and bottom of the bonded phase. The columns are placed in a Vac Elut apparatus and washed twice with 2 ml portions of hexane [Kaluzny, 1985,& Prasad, 1988. The dry lipid samples are taken up in two 0.150 ml portions of chloroform and applied to the column under atmospheric pressure. After adsorption the neutral lipids are eluted with 4 ml of chloroform-2-propanol (2:1, v/v) and the free fatty acids eluted with 4 ml of 2% acetic acid in diethyl ether. The solvent containing the free fatty acids is dried under a stream of N₂.

### GLC analysis

The dry free fatty acid residue is processed for the derivatization of methyl esters by the boron trifluoride-methanol method [Prasad 1988]. 1 ml of boron trifluoride in methanol (BF3) is added to each sample, the sample vials are heated at 70°C for 5 minutes, shaken vigorously, and baked for a further 10 minutes. Once the samples reached room temperature 0.5 ml milli Q water is added, the sample vials shaken, 0.1 ml heptane is added and the sample shaken again. 0.05 ml of the top heptane layer is removed for GLC analysis. A Model HP5890 Plus Series 2 (Hewlett-Packard) gas chromatograph equipped with a DB-225 column is used to separate the methyl esters of the fatty acids and a Model HP 5890 GC with a Model HP 5973 MS (Hewlett-Packard) GC/MS used to confirm the identity of individual free fatty acids. The temperature program consisted of a linear increase from an initial temperature of 80°C to a final temperature of 210°C at a rate of 3°C/min followed by a ten-minute period at the final temperature. The quantitation of tissue fatty acids is based on retention times of fatty acid methyl ester standards and relative theoretical response factors. Free fatty acids are assigned based on standards and on GC/MS chromatograms.

### Enzymatic analysis of tissue free fatty acids and triglyceride

Free fatty acids are separated from total lipid, evaporated down under a stream of N₂ and stored at -80°C until analysis. Samples are dissolved in 50 µl of warm ethanol (35-40 °C), 0.625 ml of a 6% Triton X-100 solution is added once the ethanol reached room temperature. The solution is stirred for 30 minutes, then made up to 0.825 ml with the Triton solution. Free fatty acids are quantified using the free fatty acids, half-micro test by Boehringer Mannheim (Germany) and the Cobas Mira (Roche Molecular Systems, New Jersey), using a palmitic acid standard. Triglyceride levels are quantified in the total lipid fraction, using the Triglyceride test by Pointe Scientific (Detroit, Michigan)and a glycerol standard.

### EXAMPLE 3

### VARIATION IN MILK COMPOSITION WITHIN INDIVIDUAL MEMBERS OF TWO FRIESIAN CATTLE HERD

This example describes analysis of milk produced by individual cows in two large Friesian diary herds. We found that significant numbers of individual cows produced milk with a low melting point (hereinafter, "Melt Pt") and a low Sold Fat Content at 10 degrees Celcius (hereinafter, "SFC10"), two measures of milk fat composition that are closely related to a reduced saturated fat content, and increased MUFA and PUFA content. Individual cows in the lowest 1-percentile, 5-percentile, or even lowest 10-percentile of the herd produce milk with extremely low saturated fat content, and high MUFA and PUFA content.

Individual milk samples were obtained from individual Friesian cows from two large dairy herds located in the Doone and Manono regions of New Zealand. Melt Pt and SFC10 were measured using standard methods. Jensen, ed., 1995, Handbook of Milk Composition. Academic Press, New York, NY; Jensen & Clark, 1988, "Lipid composition and properties," in: Wong, ed., Fundamentals of Dairy Chemistry, 3rd ed., Van Nostrand Reinhold, New York, NY, pp. 171; Fox, ed., 1995, Advanced Dairy Chemistry. Vol. 2. Lipids. 2nd Ed, Chapman and Hall, New York, NY. Melt Pt and SFC10 were determined for each individual milk sample. Results of these analyses are shown in Figures 1, 2 and 3.
Figure 1 is a bar graph showing the results of the Melt Pt measurements performed on the individual milk samples. Melt Pt is plotted on the X axis, and the number of cows within the herd possessing a particular Melt Pt is indicated on the *Y* axis. The upper and lower panels depict results from the Doone and Manono herds, respectively. The Melt Pt is closely related to, and is a measure of, the saturated fatty acid content of the milk, with lower Melt Pts indicating lower levels of saturated fatty acids, and higher Melt Pts indicating higher levels of saturated fatty acids.
Figure 2 is a bar graph showing the results of the SFC 10 testing. SFC10 is plotted on the X axis, and the number of cows within the herd possessing a particular SFC 10 is indicated on the *Y* axis. The SFC10 value is closely related to, and is a measure of, the saturated fatty acid content of the milk, with lower SFC10 values indicating lower levels of saturated fatty acids.
Figure 3 is a graph showing the Melt Pt (plotted on the *X* axis) and the SFC 10 (plotted on the *Y* axis) measurements for each individual milk sample. Each point represents milk fat from a single cow, and the upper and lower panels depict the results from the Doone and Manono herds, respectively. To examine the relationship between the Melt Pt and SFC10 measurements, regression analysis was performed using standard statistical methods. The correlation coefficients (or *r* values) between the Melt Pt and SFC10 measurements were r *=* 0.73 and *r* = 0.980 for the individual milk samples collected from the Doone and Manono herds, respectively. These "r" values indicate that there is a significant correlation between Melt Pt and SFC10 values in the individual milk samples. These results indicate that it is likely that 5-10% of the individual cows in two large dairy herds produce milk that is likely to have the preferred composition.

Although the foregoing invention has been described in some detail by way of illustration and example for purposes of clarity and understanding, it will be apparent to those skilled in the art that certain changes and modifications may be practiced. Therefore, descriptions and examples should not be construed as limiting the scope of the invention.

All patents, patent applications, and publications cited herein are hereby incorporated by reference in their entirety for all purposes to the same extent as if each individual publication, patent or patent application are specifically and individually indicated to be so incorporated by reference.

This invention may also be said to broadly consist in the parts, elements and features referred to or indicated in the specification of the application, individually or collectively, and any or all combinations of any two or more of said parts, elements or features, and where specific integers are mentioned herein which have known elements in the art to which this invention relates, such known equivalents are deemed to be incorporate herein as if individually set forth.

The invention will now be described with reference to the following numbered clauses:
1. A population of cows wherein substantially all of the milk-producing cows in the population produce milk comprising less than about 60% total saturated fat, at least about 30% mono-unsaturated fatty acids (MUFA), and at least about 9% total poly-unsaturated fatty acids (PUFA) when fed a conventional diet.
2. The population of clause 1, wherein the milk comprises less than about 60% total saturated fat, less than about 10% myristic 14:0, less than about 20% palmitic 16: 0, at least about 30% total MUFA, at least about 25% oleic 18: 1 total, at least about 6% total PUFA, and at least about 5% linoleic 18: 2.
3. The population of clause 1, wherein the cow population comprises at least 10 milk-producing cows.
4. The population of clause 3, wherein at least one of the cows is a Friesian, Guernsey, Holstein, Ayreshire, Jersey, Brown Swiss, or Milking Shorthorn.
5. A method of generating a population of cows wherein substantially all of the milk-producing cows in the population produce MFAC milk, said method comprising:
   (a) obtaining a milk sample produced by an individual cow;
   (b) determining whether the fat composition of the milk sample is characteristic of a MFAC milk;
   (c) identifying an individual cow that produced a milk sample with a fat composition characteristic of a MFAC milk as a milk-producing cow that produces MFAC milk;
   (d) repeating steps (a) to (c) with additional individual cows until a plurality of cows are identified as milk-producing cows that produce MFAC milk; and,
   (e) physically or informationally segregating the plurality of cows, thereby generating a cow population wherein substantially all of the milk-producing cows in the population produce MFAC milk.
6. The method of clause 5 wherein the fat composition characteristic of a MFAC milk is less than about 60% total saturated fat, at least about 30% mono-unsaturated fatty acids, and at least about 9% total poly-unsaturated fatty acids.
7. The method of clause 6 wherein the fat composition characteristic of a MFAC milk is less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2.
8. The method of clause 5 wherein the plurality is at least 10 cows.
9. A population of cows generated by the method of clause 5.
10. A method for breeding cattle to generate progeny cows that produce MFAC milk, said method comprising:
   (a) identifying at least one cow that, when fed a conventional diet, produces milk with a fat composition characteristic of a MFAC milk;
   (b) breeding the cow to produce progeny; and,
   (c) selecting progeny that produce milk with a milk fat composition characteristic of a MFAC milk.
11. The method of clause 10 wherein the fat composition characteristic of a MFAC milk is less than about 60% total saturated fat, at least about 30% mono-unsaturated fatty acids, and at least about 9% total poly-unsaturated fatty acids.
12. A population of cows produced according to the method of clause 11, wherein substantially all of the milk-producing cows in the population produce milk comprising less than about 60% total saturated fat, at least about 30% mono-unsaturated fatty acids (MUFA), and at least about 9% total poly-unsaturated fatty acids (PUFA) when fed a conventional diet.
13. Progeny of a cow in the population of clause 5 or clause 12.
14. A pooled milk composition comprising milk from a plurality of individual cows capable of producing MFAC milk when fed a conventional diet.
15. The composition of clause 14 wherein the plurality comprises at least 10 cows.
16. The composition of clause 14 that does not contain milk from cows that do not produce MFAC milk.
17. A pooled milk fat composition comprising milk from a plurality of individual cows fed conventional diets, wherein the pooled milk composition possesses a fat composition characteristic of the fat composition of a MFAC milk, said MFAC milk comprising less than about 60% total saturated fat, at least about 30% mono-unsaturated fatty acids (MUFA), and at least about 9% total poly-unsaturated fatty acids (PUFA).
18. A milk-based product made using the pooled milk composition of clauses 14-17.
19. The product of clause 18, wherein the milk-based product is selected from the group consisting of powdered milk, condensed milk, skim milk, cream, butter, cheese, chocolate, ice cream, yogurt and infant-formula.
20. A method of identifying an individual milk-producing cow that produces MFAC milk comprising:
   (a) obtaining a milk sample produced by an individual cow that has been fed a conventional diet for at least about three days prior to the time the sample is obtained;
   (b) determining whether the fat composition of the milk sample is characteristic of a MFAC milk; and,
   (c) identifying an individual cow that produced a milk sample with a fat composition characteristic of a MFAC milk as a milk-producing cow that produces MFAC milk.
21. The method of clause 20, further comprising repeating steps (a) to (c) with additional individual cows until a plurality of cows are identified as milk-producing cows that produce MFAC milk.
22. The method of clause 21, wherein the plurality of cows comprises at least 5 cows.
23. The method of clause 21, further comprising physically or informationally segregating the plurality of cows, thereby generating a cow population wherein substantially all of the milk-producing cows in the population produce MFAC milk.
24. The method of clauses 20-23, wherein the fat composition characteristic of a MFAC milk is less than about 60% total saturated fat, at least about 30% mono-unsaturated fatty acids, and at least about 9% total poly-unsaturated fatty acids.
25. The method of clause 24 wherein the fat composition characteristic of a MFAC milk is less than about 60% total saturated fat, less than about 10% myristic 14:0, less than about 20% palmitic 16:0, at least about 30% total MUFA, at least about 25% oleic 18:1ₜₒₜₐₗ, at least about 6% total PUFA, and at least about 5% linoleic 18:2.
26. A method of identifying an individual cow capable of producing MFAC milk, said method comprising:
   (a) identifying a genetic marker in bovines associated with the phenotype in milkproducing cows of producing MFAC milk;
   (b) obtaining a nucleic acid sample from an individual cow; and,
   (c) detecting the presence of the genetic marker in the nucleic acid, thereby identifying the identifying the cow as an a cow capable of producing MFAC milk.

## Claims

1. A method of producing MFAC milk from bovine cows capable of producing MFAC milk when fed a conventional diet, the method including the steps of:
(i) identifying bovine cows capable of producing MFAC milk when fed a conventional diet;
(ii) selecting cows capable of producing MFAC milk when fed a conventional diet; and
(iii) milking the selected cows to obtain the MFAC milk;
where the MFAC milk comprises:
(A) less than about 60% total saturated fat, at least about 30% monounsaturated fatty acids and at least about 9% total polyunsaturated fatty acids; or
(B) less than about 60% total saturated fat, at least about 30% monounsaturated fatty acids, at least about 6% total polyunsaturated fatty acids and less than about 20% palmitic (16:0) acid; or
(C) less than about 60% total saturated fat, at least about 30% monounsaturated fatty acids, at least about 6% total polyunsaturated fatty acids and at least about 5% linoleic (18:2) acid.

2. A method as claimed in claim 1 where step (i) includes:
(a) obtaining a milk sample from a bovine cow; and
(b) determining whether the fat content of the milk sample is characteristic of MFAC milk.

3. A method as claimed in claim 1 where step (i) includes:
(c) identifying a genetic marker in bovine cows or bulls, which marker is associated with a phenotype in milk-producing cows of producing MFAC milk;
(d) obtaining a nucleic acid sample from a bovine cow or bull; and
(e) detecting the presence of the genetic marker in the nucleic acid.

4. A method as claimed in any one of claims 1 to 3 where step (ii) includes physically separating bovine cows identified in step (i) from bovine cows that do not produce MFAC milk.

5. A method as claimed in any one of claims 1 to 3 where step (ii) includes informationally separating bovine cows identified in identifying step (i) from bovine cows that do not produce MFAC milk.

6. A method as claimed in any one of claims 1 to 5 where the MFAC milk has between about 50% and about 60% total saturated fat, between about 30% and about 40% monounsaturated fatty acids and between about 9% and about 11% polyunsaturated fatty acids.

7. A method as claimed in any one of claims 1 to 5 where the MFAC milk has less than about 55% total saturated fat, at least about 32% monounsaturated fatty acids and at least about 10% polyunsaturated fatty acids.

8. A method as claimed in any one of claims 1 to 5 where the MFAC milk has less than about 55% total saturated fat, at least about 32% monounsaturated fatty acids, at least about 10% polyunsaturated fatty acids and less than about 19% palmitic (16:0) acid.

9. A method as claimed in any one of claims 1 to 5 where the MFAC milk has between about 50% and about 60% total saturated fat, between about 30% and about 40% monounsaturated fatty acids, between about 6% and about 12% polyunsaturated fatty acids and between about 15% and about 20% palmitic (16:0) acid.

10. A method as claimed in any one of claims 1 to 5 where the MFAC milk has less than about 55% total saturated fat, at least about 32% monounsaturated fatty acids, at least about 10% polyunsaturated fatty acids and less than about 7% linoleic (18:2) acid.

11. A method as claimed in any one of claims 1 to 5 where the MFAC milk has between about 50% and about 60% total saturated fat, between about 30% and about 40% monounsaturated fatty acids, between about 6% and about 12% polyunsaturated fatty acids and between about 5% and about 10% linoleic (18:2) acid.

12. A method as claimed in any one of claims 6 to 11 where the monounsaturated fatty acid includes oleic (18:1) and oleic (18:1)ₜᵣₐₙₛ acid.

13. A method as claimed in any one of claims 6 to 11 where the polyunsaturated fatty acid includes two linoleic fatty acids, which are linoleic (18:2) acid and linolenic (18:3) acid.

14. A method as claimed in any one of claims 1 to 13 where the MFAC milk contains less than about 15 mg of cholesterol per 100g whole fluid milk.

15. A method as claimed in claim 14 where the MFAC milk contains less than about 13 mg of cholesterol per 100g whole fluid milk.
